# EUROPEAN PATENT SPECIFICATION

(11) **EP 3 416 526 B1**
(45) Date of publication and mention of the grant of the patent: **08.01.2020**
(21) Application number: 17705454.1
(22) Date of filing: 08.02.2017
(51) Int. Cl.: A47C 31/12, A61B 5/00, A61G 7/057, A61B 5/11

(54) **PRESSURE MONITORING CUSHION**
DRUCKÜBERWACHUNGSKISSEN
COUVERTURE DE SURVEILLANCE DE PRESSION

(30) Priority: 17.02.2016 GB 201602774
(43) Date of publication of application: 26.12.2018
(73) Proprietor: The Helping Hand Company (Ledbury) Ltd, Hereforedshire HR8 1NS (GB)
(72) Inventor: JAMES, Gavin, Ledbury Herefordshire HR8 1NA (GB); MOORE, Stuart, Colwall, Herefordshire WR13 6QP (GB)
(74) Representative: Games, Robert Harland
(86) International application number: PCT/GB2017/050306
(87) International publication number: WO 2017/141008

(56) References cited:
- WO-A1-2007/106040
- WO-A1-2015/003211
- DE-A1- 19 802 099
- US-A1- 2006 065 060
- US-A1- 2014 130 610

## Description

The present invention relates to a pressure monitoring cushion for monitoring persons who are at risk of developing pressure ulcers.

### BACKGROUND TO THE INVENTION

Patients who spend long periods of time in one position, whether in hospital or otherwise, are at risk of pressure ulcers forming. Whilst the skin can tolerate a high pressure for short period, or sustained lower pressure over a longer period, spending excessive time in one position can cause capillaries in the skin to close up, which may lead to skin necrosis and the subsequent formation of a pressure ulcer (also known as pressure sores or bedsores). Patients at risk of developing such ulcers include, for example, those who are elderly, paraplegic, tetraplegic, critically ill or terminally ill. The most commonly affected parts of the body can include the sacrum, the coccyx, the heels and the hips, although they may also form elsewhere.

Pressure ulcers can arise due to sustained pressure on an area of soft tissue, often over a bony prominence, with a range of factors potentially contributing to their formation, including but not limited to: shearing (where the skin remains static but the underlying tissues do not) and microclimate (temperature and level of moisture) between the bed and a patient's skin. Perspiration and incontinence both contribute to the skin microclimate, increasing the level of moisture and potentially accentuating the development of one or more pressure ulcers.

It can take many weeks for a pressure ulcer to completely heal, with more advanced ulcers potentially never fully healing, although the age and general state of health of the patient will also affect the outcome. They are painful at all stages for all patients, and increase the likelihood of infection for the immunocompromised.

The early identification of pressure ulcers can greatly increase the chance of successful treatment, and although the technique of regularly turning bedridden patients has long been known to help reduce their prevalence, this requires nursing staff, for example, to adhere to a regular schedule of turning multiple patients.

Maintaining a healthy diet can also minimise the degree to which pressure sores affect patients, as can effective management of the skin microclimate.

Currently, some systems use pressure sensors to detect whether a person is sat on a seat or in bed, for example, which can further be used to identify if they have left the seat or bed, or possibly fallen off. Other systems monitor the specific pressure under a person on a seat or bed.

It is an objective of the present invention to provide an improved means for reducing or substantially obviating the aforementioned problems.

### STATEMENT OF INVENTION

According to the present invention, there is provided a pressure monitoring cushion comprising first and second pressure sensors, a controller configured to receive input from the pressure sensors, output means controlled by the controller, and a power source, wherein
each pressure sensor includes first and second electrical conductors and a resilient electrical insulator disposed between the electrical conductors;
each electrical insulator includes one or more apertures through which the adjacent electrical conductors come into contact when a threshold pressure is applied to the cushion;
the electrical conductors of the first pressure sensor come into contact at a first threshold pressure, and the electrical conductors of the second pressure sensor come into contact at a higher second threshold pressure; and
the controller triggers an output from the output means after a predetermined period of time has elapsed with substantial contact between the electrical conductors in the first and/or second pressure sensors.

The pressure monitoring cushion supports one or more parts of the body, potentially reducing the risk of the formation of pressure ulcers by redistributing pressure evenly. The whole cushion acts as a dual pressure sensor. Normal pressure is detected via the first pressure sensor and excess pressure is detected via the second pressure sensor. The pressure sensors are essentially pressure switches which activate at different pressure thresholds, corresponding to acceptable and unacceptable levels of pressure on the body of a person using the cushion. The second pressure sensor may be considered to function as a time-dependent pressure switch.

Sitting on the cushion compresses the resilient insulating layers, such that the conductors can come into contact via the aperture(s) and complete an electrical circuit. The controller then starts timing and, after a set period has passed without loss of contact between the conductors, initiates the output means. This alerts the person sitting on the cushion (or their carer) that they have been in the same position for a long time and may be at risk of developing a pressure ulcer, prompting re-positioning.

The period of time may be customised prior to use of the cushion. This is because a reduced delay period may be appropriate for an elderly person as opposed to a younger person, as they are more likely to sustain injury easily. For example, the period of time from initial use to activation of the output means could be set to 3 hours, 30 minutes, or a period of time in between these.

When the second pressure sensor is brought into operation with the first pressure sensor, detecting the second threshold pressure, then the controller may shorten the period of time until the output means is triggered.

The second pressure sensor is brought into operation when its conductors come into contact. This happens when a person is applying a particularly high pressure to the cushion, usually through a bony prominence. The controller recognises the change in state of the second pressure sensor, and reduces the scheduled time until the output means is triggered. This alerts the user (or their carer) to move/re-position earlier than otherwise scheduled, mitigating the risk of a pressure ulcer developing. Operation of the second pressure sensor may also indicate that the cushion is deteriorating, if the insulating layer is more readily allowing the conductors to touch. Since this gives reduced support, the earlier alert can mean that the cushion is worn out and needs replacing/refurbishment.

The output means may issue a first output when the electrical conductors of the first pressure sensor are brought into contact. The output means may issue a different second output when the respective electrical conductors of the first and second pressure sensors are brought into contact. The contact between electrical conductors may be regarded as closure of a switch.

After sitting on the cushion, the output means is activated to confirm whether the cushion is operating correctly. The first output signals that only the first pressure sensor is in operation, i.e. the electrical conductors of the first pressure sensor are in contact, and so the level of pressure is within an acceptable range. However, if both pressure sensors are in operation, i.e. both sets of electrical conductors are in respective contact, a second output is issued. This means that the level of pressure when a person has begun to use the cushion is unacceptable. This may indicate that the person is awkwardly positioned, the cushion is unsuitable for the weight of the person using the cushion, or that the cushion is deteriorating or has undergone structural failure. In either case, the second output is indicative of inadequate support and remedial action can be taken.

At least one property of the first and/or second pressure sensors may be calibrated for a user, for example, according to their weight and/or body shape. The resilient electrical insulator of the second pressure sensor may have a greater depth than the resilient electrical insulator of the first pressure sensor. The resilient electrical insulator of the second pressure sensor may be more resistant to deformation than the resilient electrical insulator of the first pressure sensor.

By customising the stiffness and/or thickness of the cushion materials, the pressure sensors can be customised to suit different ranges of user weight and/or body shape. For example, heavier patients may need cushions that deform less under pressure, whilst lighter patients (e.g. young children) may need cushions that deform to a greater extent, so that the pressure sensors operate and initiate alerts appropriately. Varying the stiffness and/or thickness gives a large range of options in adjusting the pressure thresholds of the cushion to suit a particular patient.

The first and second pressure sensors may be stacked within the cushion. The first and second pressure sensors may have a common electrical conductor between their resilient electrical insulators.

By using the pressure sensors in a stack, they function in series. Therefore, higher pressure is needed to operate whichever pressure sensor is lower in the stack, since more material must be deformed to bring the corresponding electrical conductors into contact. Using a common or shared electrical conductor (i.e. the second electrical conductor of the first pressure sensor is the first electrical conductor of the second pressure sensor) can make the device more sensitive to pressure, reducing the difference between the first and second pressure thresholds.

The controller may be programmed to disable or suppress the output means at predetermined times.

This can be used to prevent the alert being activated during the night, for example, when a person using the cushion will typically be asleep. Alternatively, a quieter alert may be issued to avoid disturbing other patients in a communal ward, for example.

The controller may be programmed to reset the period of time that has elapsed since the electrical conductors came into contact if the electrical conductors lose contact for an interval. Preferably, the interval is substantially 0.5 seconds or longer.

Advantageously, this can account for a person shifting their own weight to a new position, even without the knowledge of a carer. This prevents the carer inadvertently returning a patient to their original position after too little time since adopting that position, and also prevents the cushion activating an alert unnecessarily if the person is moving sufficiently often on their own. It also accounts for the person getting up from the seat where the cushion is located.

Each electrical conductor may include a flexible material. Each electrical conductor may include a carbon-printed surface. Preferably, each electrical conductor includes carbon-printed polythene.

Under pressure, the electrical conductors in a given pressure sensor can enter one or more of the apertures in the electrical insulator, contacting the opposing conductor. For example, the conductors may include a fabric or other material which can flow (or slip) into the apertures during use. It is safer to use conductors with carbon-printed surfaces, as opposed to metal for example, because this prevents electrical arcing within the cushion if the conductors are proximal but not in contact. Carbon-printed surfaces are also more durable than metal counterparts over repeated cycles of compression. Carbon-printed polythene is particularly durable and flexible.

Each resilient electrical insulator may include foam, or a substantially foam portion.

Using a foam electrical insulator makes the cushion more comfortable to sit or lie on. The foam may also be compressed into a significantly smaller volume under pressure, shortening the distance the electrical conductors must bridge to make contact. Optionally, using foam with a honeycomb structure improves ventilation relative to a solid layer of equivalent thickness, whilst still being structurally resilient.

The output means may include a speaker, and when the period of time has elapsed, instructions are issued via the speaker for a person sitting on the cushion or their carer. Preferably, the content and language of the instructions is customisable. For example, a pre-recorded voice message may be provided saying "Please sit in a new position". The output means may include a visual indicator for signalling that the period of time has elapsed.

This can alert the person sat on the cushion or their carer that they need to shift their weight to avoid developing a pressure ulcer. An audible alert is particularly useful if the person using the cushion is blind or has poor eyesight, or merely has their eyes closed. Having the option to set a chosen language is beneficial in a hospital, for example, where different patients speak different languages. This conveys instructions in a manner understood by the individual user, in case nursing staff are unable to attend to them in good time. A visual alert is particularly useful if the person using the cushion is deaf or hard of hearing, even if a hearing aid is being used.

The output means may include a signal for communication with an external device. Preferably, the signal is transmitted wirelessly for receipt by the external device.

Advantageously, the signal can generate an alert accessible via the external device (e.g. a pager, a phone, etc.), so that a carer is alerted even when located at a distance from their patient. A wireless signal is useful where the carer is located at larger distances from the patient. The signal may be (or may trigger) any form of alert or notification which conveys that the output means of the cushion has been activated. The signal may be sent to multiple external devices, so that the nearest or next available carer can attend to the patient.

The pressure monitoring cushion may be incorporated into or used with one of the following: a bed; a mattress; a seat; a chair; a wheelchair.

### BRIEF DESCRIPTION OF THE DRAWINGS

For a better understanding of the present invention, and to show more clearly how it may be carried into effect, reference will now be made by way of example only to the accompanying drawings, in which:
Figure 1 shows an exploded perspective view of a first embodiment of a pressure monitoring cushion;
Figure 2 shows an exploded perspective view of a second embodiment of a pressure monitoring cushion;
Figure 3 shows a cross-sectional view of the pressure monitoring cushion of Figure 2 in use, where the uppermost pressure sensor is in operation; and
Figure 4 shows a cross-sectional view of the pressure monitoring cushion of Figure 2 in use, where the upper and lower pressure sensors are both in operation.

### DESCRIPTION OF PREFERRED EMBODIMENTS

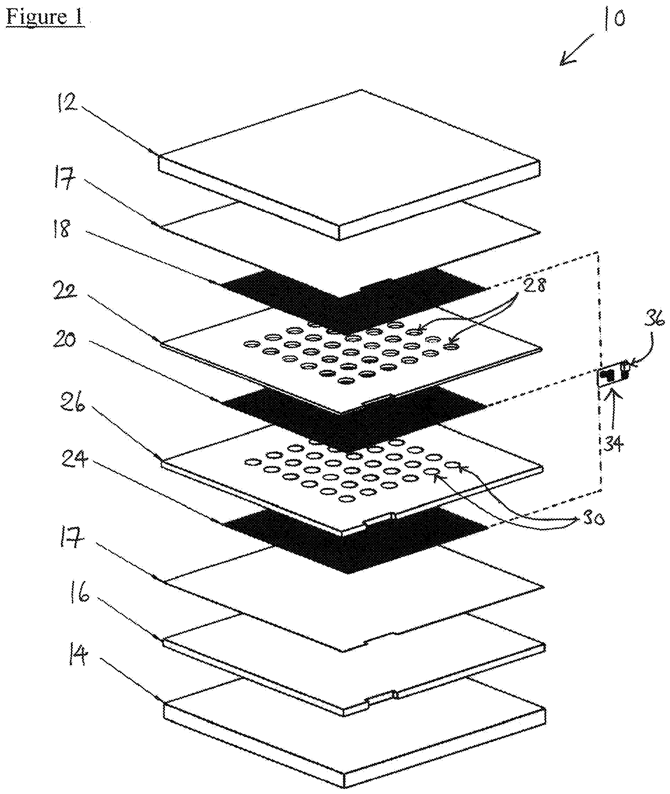

Referring to Figure 1, a first embodiment of a pressure monitoring cushion is indicated generally at 10. The cushion 10 includes a top cushion 12 and a bottom cushion 14. Another cushioned layer 16 is provided adjacent the bottom cushion 14. The top and bottom cushions 12, 14 are the same thickness in this embodiment, with the cushioned layer 16 being about half as thick as these. This corresponds to a depth of 25 mm for the cushions 12, 14 and 12 mm for the cushioned layer 16. The materials used to construct the cushion 10 are selected to ensure that regular washing will not cause degradation of the device or impede its functionality.

First and second pressure sensors are provided in the cushion 10. The first and second pressure sensors are disposed in different horizontal planes in the cushion 10. In this embodiment, the pressure sensors are between the cushions 12, 14. The pressure monitors are also stacked one above the other within the cushion 10. In this embodiment, the first pressure sensor lies above the second pressure sensor in the cushion 10. The pressure sensors act as pressure switches. Fabric sheets 17 are provided between the top cushion 12 and the cushioned layer 16, about the first and second pressure sensors.

The first pressure sensor includes two electrical conductors (or conductive sheets) 18, 20, and a resilient electrical insulator (or insulating layer) 22 disposed between the sheets 18, 20. The second pressure sensor also includes two conductive sheets 20, 24, and an insulating layer 26 disposed between the sheets 20, 24. In this embodiment, the pressure sensors share a common conductive sheet 20. However, other embodiments may have distinct conductive sheets for each pressure sensor. These conductive sheets (of different pressure sensors) may be separated by an insulating layer to prevent contact between them.

Each insulating layer 22, 26 is made of resilient foam, providing a cushioning effect in addition to the other cushions 12, 14, 16. In some embodiments, the foam is CMHR foam (Combustion Modified High Resilience foam), so its fire resistance properties are enhanced and it meets relevant Fire Safety Regulations.

Applying pressure to cushion 10 compresses the insulating layers 22, 26 and allows the conductive sheets 18, 20, 24 to approach each other more closely. It can be seen from Figure 1 that the lower insulating layer 26 is around twice as thick as the upper insulating layer 22 in this embodiment. In this embodiment, the layers are 10 mm and 5 mm thick respectively. Therefore, the lower insulating layer is more difficult to compress. The actual thicknesses can be calibrated accordingly to the weight of the intended user. Overall, this gives the second pressure sensor a higher effective pressure threshold before its conductive sheets 20, 24 can come into contact, relative to the pressure threshold of the first pressure sensor.

Each insulating layer 22, 26 includes a plurality of apertures or holes 28, 30. The holes 28, 30 extend from one side of the respective layer 22, 26 to the other. The holes 28, 30 are spaced evenly in a 6x6 square (partially shown) in this embodiment, but may be arranged regularly or irregularly in other embodiments, with higher or lower numbers of apertures than in the present embodiment. For example, there may be 8x6, 8x8, 10x8 or other arrangements of holes, where a rectangular layout is used. There may be a higher number of holes where higher pressure is expected to be applied in use, such as towards a central region of the cushion, for example. The holes may have different cross-sectional areas. The holes may have different cross-sectional shapes. The shape and or area of the cross-section may be selected based on the expected distortion under pressure in use.

The holes 28 in the upper insulating layer 22 are co-aligned with the holes 30 in the lower insulating layer 26. In other embodiments, the holes in different layers may be anti-aligned, or have another alignment. The insulating layers 22, 26 are compressed when the cushion 10 is sat upon, but the apertures 28, 30 substantially retain their size, shape and relative spacing in use. The conductive sheets 18, 20, 24 cover the apertures 28, 30 on the respective sides of the insulating layers 22, 26.

Each conductive sheet 18, 20, 24 is a carbon-printed (or carbon-coated) sheet of polythene. The polythene is durable and can undergo deformation into the holes 28, 30 over many cycles without wearing out. Using carbon as a conductor is better than metal because it is lighter, it will not undergo metal fatigue during repeated compression cycles, and internal arcing across air gaps will not occur if a small gap is present between the conductive sheets 18, 20, 24.

A controller 34 is connected to the conductive sheets 18, 20, 24. In this embodiment, an LED 36 is used to visually indicate excess pressure. Side portions of parts of the cushion 10 are cut-out to accommodate the controller 34 at the side of the cushion 10.

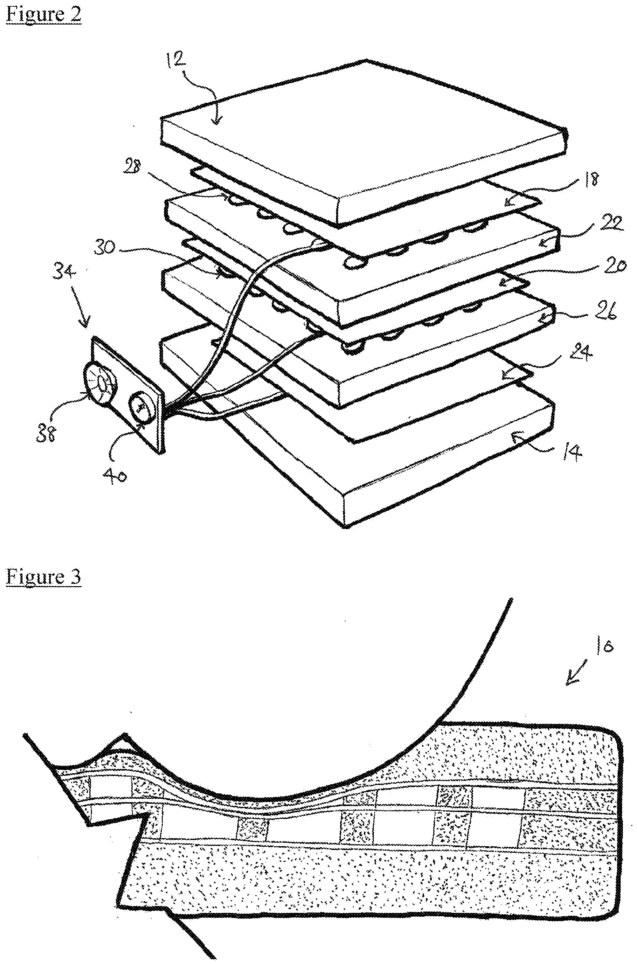

A power source or energy source (not shown) for powering the controller 34 and LED 36 is also provided. The battery is mounted behind the controller 34 as viewed in Figure 2. Together, the controller 34, conductive sheets 18, 20, 24, LED 36 and energy source form an electrical circuit. In this embodiment, the power source is a battery. The controller 34 is programmed to issue a warning signal via the output (here, LED 36) in the event that the battery charge is low.

The controller 34 includes a processor (not shown) which controls activation of the LED 36 when a pre-set period of time (or delay period) has elapsed. In other words, the controller 34 delays activation of the LED 36 if the conductive sheets 18, 20 of the first pressure sensor come into contact. The pre-set period is measured from the moment at which the conductive sheets 18, 20 of at least the first pressure sensor last came into contact. 'Contact' means to sustained contact between the conductive sheets 18, 20, 24, i.e. where any loss of contact lasts less than 0.5 seconds, with reference to the interval below.

In this embodiment, the pre-set period can be adjusted using a variable control (not shown), taking account of the condition of the user. The period may be set prior to using the cushion to 30 minutes, 3 hours, or any length of time between these. The variable control is a rotary switch which is adjustable using a screwdriver (or similar), preventing accidental changes to the pre-set period. It will be appreciated that the LED 36 could be normally 'on', and delayed deactivation could be an alert instead.

The controller 34 is also adapted to reset the delay period to its initial value if the person shifts their weight before the delay period has expired, i.e. if the patient moves and eases pressure on the skin, allowing blood to flow and reach the area which was under pressure. This is identified by monitoring whether the conductive sheets 18, 20 of the uppermost pressure sensor move out of contact for an interval substantially 0.5 seconds or more in length. In other embodiments, the interval (or delay period) may be linked to the second pressure sensor. The interval may also be shorter or longer as needed in alternate embodiments.

The controller is programmed to recognise when the second pressure sensor is 'closed', i.e. the conductive sheets 20, 24 are in contact. Since the first pressure sensor is already closed when this occurs, excess pressure is determined to be present. When the second and first pressure sensors are both 'closed', the controller is programmed to reduce the time until the output (i.e. the LED 36) activates, as measured from the last contact initiated between the conductive sheets 18, 20. The time reduction may be proportional (e.g. a 50% reduction in remaining time) or absolute (e.g. a reduction of 45 minutes). Other values may be selected for shortening the period until output activation.

Referring now to Figure 2, a second embodiment of a pressure monitoring cushion is shown. Many of the features are the same as those of the first embodiment, and are labelled with the same reference numerals. Only features unique to the second embodiment are discussed below.

The controller 34 includes a speaker 38 and a processor (not shown). A representation of a clock 40 is also shown. The processor is programmed to deactivate the speaker 38 at night. In other words, between the hours of 2200-0700, for example, the speaker will remain silent even if it would normally otherwise sound an alert. Fabric sheets are not provided between the cushions and pressure sensors in this embodiment.

The processor includes programmed verbal instructions which are played through the activated speaker 38. In this embodiment, the controller stores instructions of approximately 2 seconds in length. The instructions are self-explanatory and tell the person atop the cushion to re-position themselves, or tell the carer the same, if the person is incapacitated for example. The instructions are recorded in different languages, so that the user can set the instruction language to one they understand.

The processor is also programmed to activate the speaker 38 on beginning to use the cushion. Once a person has sat on the cushion, the speaker 38 issues a tone (e.g. long beep) to indicate that it is operational. This corresponds to the conductive sheets 18, 20 of only the first pressure sensor being brought into contact. However, if sitting on the cushion brings both sets of conductive sheets 18, 20, 24 of the first and second pressure sensors into contact, a different tone (e.g. short beep, or a two-tone beep) is sounded to indicate that support is inadequate. This can mean that the cushion is worn out, or that the cushion is not robust enough to properly support the person using it.

In this embodiment, the electronics of the cushion are battery-powered. The controller 34 includes a PCB (printed circuit board, not shown) and the speaker 38 is PCB-mounted. The controller 34 activates the speaker 38 to issue a warning sound if the battery charge is low. The warning is periodic (or intermittent), similar to that of a smoke alarm.

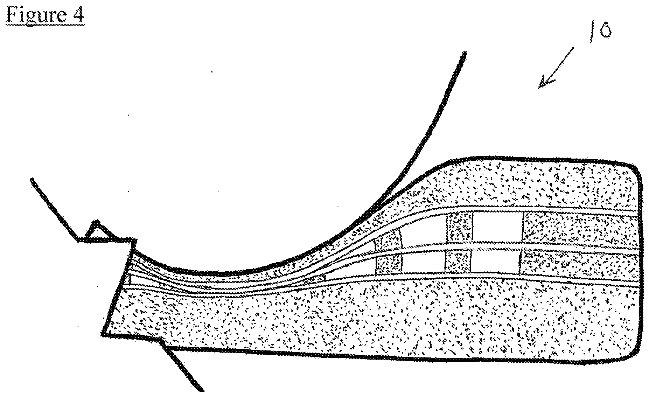

Referring now to Figures 3 and 4, the pressure sensors are shown in use. In Figure 3, a person sat on the cushion 10 has compressed the first pressure sensor and its conductive sheets 18, 20 are touching. This corresponds to normal pressure being exerted on seated parts of that person's body. No remedial action is needed until the controller initiates the relevant output device.

In Figure 4, a person sat on the cushion 10 has compressed both the first and second pressure sensors, connecting all of the conductive sheets 18, 20, 24. This corresponds to excessively high pressure being exerted on seated parts of that person's body. Remedial action may be needed soon, or immediately, to prevent increased risk of pressure ulcer formation.

In use, it is envisaged that the cushion 10 will be used beneath a person in a chair or a bed, where the person may be a hospital patient, for example, who is confined to a sitting or lying down position. The cushion 10 may be independent of the chair, bed or other element of furniture, or it may be integrated into (i.e. built into) the relevant item. Where the need exists to monitor multiple areas of the body that are separated by a distance greater than the size of the cushion 10, auxiliary monitoring cushions can be utilised alongside the primary cushion 10. In general, the cushion 10 would most likely be placed beneath the head, torso (including the hips) or feet of a patient, since these general areas support the body most when lying down.

When a patient is atop the cushion 10, the pressure exerted by the patient slowly causes the uppermost conductive sheet 18 to enter one or more of the holes 28 in the upper insulating layer 22. Once contact is made between the conductive sheets 18, 20 in the first pressure sensor, the controller 34 begins a countdown until activation of the output (whether the speaker 38, the LED 36, or another output). If pressure is redistributed to another area of the cushion 10 (and hence to another area of the patient's skin) by, for example, the natural movement of the patient in shifting their weight occasionally, then the delay timer will reset and start anew. This is because the shift in weight will disconnect the conductive sheets 18, 20, 24 for more than 0.5 seconds, resetting the delay period.

If the pressure is not redistributed to another area of the cushion 10 then the delay period will expire and the output device will activate. This warns the patient and/or their carer(s) that the patient has not changed position significantly over the course of the delay period and is at risk of developing a pressure ulcer, and hence attention is needed. After re-positioning, the cushion 10 continues to monitor the patient in their new position in the same manner as when monitoring their first seated position.

Other embodiments are envisaged within the scope of the claims. For example, the first pressure sensor may be divided into two distinct halves within the upper portion of the cushion. In other words, the first pressure sensor would be a dual zone pressure sensor. A patient could be positioned above one half of the first pressure sensor and, when the relevant time period had elapsed, could be turned to lie above the other half of the first pressure sensor, i.e. on the other side of the cushion. The controller would be able to distinguish between activation of either half of the first pressure sensor and reset the timing circuit accordingly.

Further embodiments may have the cushion integrated into a car seat or a wheelchair. The electrical conductors may be composed of electrically conductive fibres as a mesh, for example. Additional pressure sensors may be provided, with greater, lesser or intermediary thickness or firmness, for example, giving a wider range of potential pressure thresholds for monitoring a patient. The controller can include a signal generator which activates after the delay period ends, sending a wireless signal to an external device. The external device receiving the signal may be a pager, a phone or a computer, for example, alerting a carer that a patient should be turned. In a further embodiment, the signal may be sent non-wirelessly.

The cushion therefore provides an inexpensive means of detecting pressure above two different thresholds, where the first threshold corresponds to acceptable pressure for a given period of time, and the second (higher) threshold corresponds to unacceptable pressure which can only be tolerated for a shorter period of time. Re-positioning or shifting the body of a person on the cushion allows blood flow to be restored and the areas of the body that were under pressure can recover, reducing the risk of a pressure ulcer forming in those areas.

The embodiments described above are provided by way of example only, and various changes and modifications will be apparent to persons skilled in the art without departing from the scope of the present invention as defined by the appended claims.

## Claims

1. A pressure monitoring cushion (10) comprising first and second pressure sensors, a controller (34) configured to receive input from the pressure sensors, output means (36, 38) controlled by the controller (34), and a power source, wherein
each pressure sensor includes first and second electrical conductors (18, 20, 24) and a resilient electrical insulator (22, 26) disposed between the electrical conductors (18, 20, 24);
each electrical insulator (22, 26) includes one or more apertures (28, 30) through which the adjacent electrical conductors (18, 20, 24) come into contact when a threshold pressure is applied to the cushion (10);
the electrical conductors (18, 20) of the first pressure sensor come into contact at a first threshold pressure, and the electrical conductors (20, 24) of the second pressure sensor come into contact at a higher second threshold pressure; and
the controller (34) triggers an output from the output means (36, 38) after a predetermined period of time has elapsed with contact between the electrical conductors (18, 20, 24) in the first and/or second pressure sensors;
***characterised in that*** the controller (34) shortens the period of time until the output means (36, 38) is triggered when the second pressure sensor detects the second threshold pressure.

2. A pressure monitoring cushion (10) as claimed in claim 1, in which the output means (36, 38) issues a first output when the electrical conductors (18, 20) of the first pressure sensor are brought into contact, and the output means (36, 38) issues a different second output when the respective electrical conductors (18, 20, 24) of the first and second pressure sensors are brought into contact.

3. A pressure monitoring cushion (10) as claimed in claim 1 or 2, in which at least one property of the first and/or second pressure sensors can be calibrated for a particular user.

4. A pressure monitoring cushion (10) as claimed in any preceding claim, in which the resilient electrical insulator (26) of the second pressure sensor has a greater depth than the resilient electrical insulator (22) of the first pressure sensor.

5. A pressure monitoring cushion (10) as claimed in any preceding claim, in which the resilient electrical insulator (26) of the second pressure sensor is more resistant to deformation than the resilient electrical insulator (22) of the first pressure sensor.

6. A pressure monitoring cushion (10) as claimed in any preceding claim, in which the first and second pressure sensors are stacked within the cushion (10).

7. A pressure monitoring cushion (10) as claimed in any preceding claim, in which the first and second pressure sensors have a common electrical conductor (20) between their resilient electrical insulators (22, 26).

8. A pressure monitoring cushion (10) as claimed in any preceding claim, in which the controller (34) is programmed to disable or suppress the output means (36, 38) at predetermined times.

9. A pressure monitoring cushion (10) as claimed in any preceding claim, in which the controller (34) is programmed to reset the period of time that has elapsed since the electrical conductors (18, 20, 24) came into contact if the electrical conductors (18, 20, 24) lose contact for an interval.

10. A pressure monitoring cushion (10) as claimed in any preceding claim, in which each resilient electrical insulator (22, 26) includes foam.

11. A pressure monitoring cushion (10) as claimed in any preceding claim, in which the output means includes a speaker (38), and when the period of time has elapsed, instructions are issued via the speaker (38) for a person sitting on the cushion (10) or their carer.

12. A pressure monitoring cushion (10) as claimed in claim 11, in which the language of the instructions is customisable.

13. A pressure monitoring cushion (10) as claimed in any preceding claim, in which the output means includes a visual indicator (36) for signalling that the period of time has elapsed.

14. A pressure monitoring cushion (10) as claimed in any preceding claim, in which the output means (36, 38) includes a signal for communication with an external device.

15. A pressure monitoring cushion (10) as claimed in any preceding claim, incorporated into or used with one of the following: a bed; a mattress; a seat; a chair; a wheelchair.

## Patentansprüche

1. Drucküberwachungskissen (10), umfassend erste und zweite Drucksensoren, eine Steuerung (34), die konfiguriert ist, um Input von den Drucksensoren zu erhalten, Outputmittel (36, 38), die von der Steuerung (34) gesteuert werden, und eine Leistungsquelle, wobei
jeder Drucksensor erste und zweite elektrische Leiter (18, 20, 24) und eine elastisch verformbare Isolierung (22, 26) einschließt, die zwischen den elektrischen Leitern (18, 20, 24) angeordnet ist;
jede elektrische Isolierung (22, 26) eine oder mehrere Öffnungen (28, 30) einschließt, durch die die benachbarten elektrischen Leiter (18, 20, 24) in Kontakt kommen, wenn ein Schwellendruck auf das Kissen (10) angewendet wird;
die elektrischen Leiter (18, 20) des ersten Drucksensors bei einem ersten Schwellendruck in Kontakt kommen und die elektrischen Leiter (20, 24) des zweiten Drucksensors bei einem höheren zweiten Schwellendruck in Kontakt kommen; und
die Steuerung (34) einen Output vom Outputmittel (36, 38) auslöst, nachdem ein vorbestimmter Zeitraum mit Kontakt zwischen den elektrischen Leitern (18, 20, 24) im ersten und/oder zweiten Drucksensor abgelaufen ist;
**dadurch gekennzeichnet, dass** die Steuerung (34) den Zeitraum verkürzt, bis das Outputmittel (36, 38) ausgelöst wird, wenn der zweite Drucksensor den zweiten Schwellendruck nachweist.

2. Drucküberwachungskissen (10) nach Anspruch 1, wobei das Outputmittel (36, 38) einen ersten Output ausgibt, wenn die elektrischen Leiter (18, 20) des ersten Drucksensors in Kontakt gebracht werden, und das Outputmittel (36, 38) einen verschiedenen zweiten Output ausgibt, wenn die entsprechenden elektrischen Leiter (18, 20, 24) des ersten und zweiten Drucksensors in Kontakt gebracht werden.

3. Drucküberwachungskissen (10) nach einem der vorstehenden Ansprüche 1 und 2, wobei mindestens eine Eigenschaft des ersten und/oder zweiten Drucksensors für einen bestimmten Benutzer kalibriert werden kann.

4. Drucküberwachungskissen (10) nach einem der vorstehenden Ansprüche, wobei die elastisch verformbare elektrische Isolierung (26) des zweiten Drucksensors eine größere Tiefe als die elastisch verformbare elektrische Isolierung (22) des ersten Drucksensors aufweist.

5. Drucküberwachungskissen (10) nach einem der vorstehenden Ansprüche, wobei die elastisch verformbare elektrische Isolierung (26) des zweiten Drucksensors widerstandsfähiger gegen Verformung als die elastisch verformbare elektrische Isolierung (22) des ersten Drucksensors ist.

6. Drucküberwachungskissen (10) nach einem der vorstehenden Ansprüche, wobei der erste und zweite Drucksensor innerhalb des Kissens (10) gestapelt sind.

7. Drucküberwachungskissen (10) nach einem der vorstehenden Ansprüche, wobei der erste und zweite Drucksensor einen gemeinsamen elektrischen Leiter (20) zwischen ihren elastisch verformbaren elektrischen Isolierungen (22, 26) aufweisen.

8. Drucküberwachungskissen (10) nach einem der vorstehenden Ansprüche, wobei die Steuerung (34) programmiert ist, um das Outputmittel (36, 38) zu vorbestimmten Zeiten auszuschalten oder zu unterdrücken.

9. Drucküberwachungskissen (10) nach einem der vorstehenden Ansprüche, wobei die Steuerung (34) programmiert ist, um den Zeitraum zurückzusetzen, der abgelaufen ist, seit die elektrischen Leiter (18, 20, 24) in Kontakt gekommen sind, wenn die elektrischen Leiter (18, 20, 24) den Kontakt während eines Intervalls verlieren.

10. Drucküberwachungskissen (10) nach einem der vorstehenden Ansprüche, wobei jede elastisch verformbare elektrische Isolierung (22, 26) Schaum einschließt.

11. Drucküberwachungskissen (10) nach einem der vorstehenden Ansprüche, wobei das Outputmittel einen Lautsprecher (38) einschließt, und wenn der Zeitraum abgelaufen ist, über den Lautsprecher Anweisungen (38) an eine Person, die auf dem Kissen (10) sitzt, oder ihrem Betreuer ausgegeben werden.

12. Drucküberwachungskissen (10) nach Anspruch 11, wobei die Sprache der Anweisungen individualisierbar ist.

13. Drucküberwachungskissen (10) nach einem der vorstehenden Ansprüche, wobei das Outputmittel eine visuelle Anzeige (36) einschließt, um zu signalisieren, dass der Zeitraum abgelaufen ist.

14. Drucküberwachungskissen (10) nach einem der vorstehenden Ansprüche, wobei das Outputmittel (36, 38) ein Signal zur Kommunikation mit einer externen Vorrichtung einschließt.

15. Drucküberwachungskissen (10) nach einem der vorstehenden Ansprüche, das in eines der Folgenden integriert ist oder damit verwendet wird: ein Bett; eine Matratze; einen Sitz; einen Stuhl; einen Rollstuhl.

## Revendications

1. Coussin de surveillance de pression (10) comprenant des premier et deuxième capteurs de pression, un dispositif de commande (34) configuré pour recevoir un signal d'entrée des capteurs de pression, un moyen de sortie (36, 38) commandé par le dispositif de commande (34), et une source d'énergie, dans lequel
chaque capteur de pression inclut des premier et deuxième conducteurs électriques (18, 20, 24) et un isolant électrique déformable élastiquement (22, 26) disposé entre les conducteurs électriques (18, 20, 24) ;
chaque isolant électrique (22, 26) comprend une ou plusieurs ouvertures (28, 30) à travers lesquelles les conducteurs électriques adjacents (18, 20, 24) entrent en contact lorsqu'une pression seuil est appliquée au coussin (10) ;
les conducteurs électriques (18, 20) du premier capteur de pression entrent en contact à une première pression seuil, et les conducteurs électriques (20, 24) du deuxième capteur de pression entrent en contact à une deuxième pression seuil ; et
le dispositif de commande (34) déclenche une sortie du moyen de sortie (36, 38), après l'écoulement d'une période de temps prédéterminée avec le contact entre les conducteurs électriques (18, 20, 24) dans les premier et/ou deuxième capteurs de pression ;
**caractérisé en ce que** le dispositif de commande (34) raccourcit la période de temps jusqu'au déclenchement du moyen de sortie (36, 38) lorsque le deuxième capteur de pression détecte la deuxième pression seuil.

2. Coussin de surveillance de pression (10) selon la revendication 1, dans lequel le moyen de sortie (36, 38) délivre une première sortie lorsque les conducteurs électriques (18, 20) du premier capteur de pression sont mis en contact, et le moyen de sortie (36, 38) délivre une deuxième sortie différente lorsque les conducteurs électriques respectifs (18, 20, 24) des premier et deuxième capteurs de pression sont mis en contact.

3. Coussin de surveillance de pression (10) selon l'une quelconque des revendications 1 et 2, dans lequel au moins une propriété des premier et/ou deuxième capteurs de pression peut être étalonnée pour un utilisateur particulier.

4. Coussin de surveillance de pression (10) selon l'une quelconque des revendications précédentes, dans lequel l'isolant électrique déformable élastiquement (26) du deuxième capteur de pression a une profondeur supérieure à celle de l'isolant électrique déformable élastiquement (22) du premier capteur de pression.

5. Coussin de surveillance de pression (10) selon l'une quelconque des revendications précédentes, dans lequel l'isolant électrique déformable élastiquement (26) du deuxième capteur de pression est plus résistant à la déformation que l'isolant électrique déformable élastiquement (22) du premier capteur de pression.

6. Coussin de surveillance de pression (10) selon l'une quelconque des revendications précédentes, dans lequel les premier et deuxième capteurs de pression sont empilés dans le coussin (10).

7. Coussin de surveillance de pression (10) selon l'une quelconque des revendications précédentes, dans lequel les premier et deuxième capteurs de pression ont un conducteur électrique commun (20) entre leurs isolants électriques déformables élastiquement (22, 26).

8. Coussin de surveillance de pression (10) selon l'une quelconque des revendications précédentes, dans lequel le dispositif de commande (34) est programmé pour désactiver ou supprimer le moyen de sortie (36, 38) à des moments prédéterminés.

9. Coussin de surveillance de pression (10) selon l'une quelconque des revendications précédentes, dans lequel le dispositif de commande (34) est programmé pour remettre à zéro la période de temps qui s'est s'écoulée depuis le moment où les conducteurs électriques (18, 20, 24) sont entrés en contact si les conducteurs électriques (18, 20, 24) perdent le contact pendant un intervalle.

10. Coussin de surveillance de pression (10) selon l'une quelconque des revendications précédentes, dans lequel chaque isolant électrique déformable élastiquement (22, 26) comprend de la mousse.

11. Coussin de surveillance de pression (10) selon l'une quelconque des revendications précédentes, dans lequel le moyen de sortie comprend un haut-parleur (38), et lorsque la période de temps s'est écoulée, des instructions sont délivrées via le haut-parleur (38) pour une personne assise sur le coussin (10) ou son soignant.

12. Coussin de surveillance de pression (10) selon la revendication 11, dans lequel la langue des instructions est modifiable.

13. Coussin de surveillance de pression (10) selon l'une quelconque des revendications précédentes, dans lequel le moyen de sortie inclut un indicateur visuel (36) pour signaler que la période de temps s'est écoulée.

14. Coussin de surveillance de pression (10) selon l'une quelconque des revendications précédentes, dans lequel le moyen de sortie (36, 38) inclut un signal de communication avec un dispositif externe.

15. Coussin de surveillance de pression (10) selon l'une quelconque des revendications précédentes, incorporé dans ou utilisé avec l'un de ce qui suit : un lit ; un matelas ; un siège ; une chaise ; une chaise roulante.
